# EUROPÄISCHE PATENTSCHRIFT

(11) **EP 0 406 850 B1**
(45) Veröffentlichungstag und Bekanntmachung des Hinweises auf die Patenterteilung: **16.02.1994**
(21) Anmeldenummer: 90112805.8
(22) Anmeldetag: 05.07.1990
(51) Int. Cl.: C07K 3/20

(54) **Verfahren zur Herstellung von Purpurmembran enthaltend Bacteriorhodopsin**
Process for obtaining purple membranes containing bacteriorhodopsin
Procédé pour obtenir des membranes pourpres contenant de la bactériorhodopsine

(30) Priorität: 05.07.1989 DE 3922133
(43) Veröffentlichungstag der Anmeldung: 09.01.1991
(73) Patentinhaber: Consortium für elektrochemische Industrie GmbH, D-81379 München (DE)
(72) Erfinder: Neumann, Stefan, Dr. Dipl.-Biol., D-8261 Kastl (DE); Leigeber, Horst, D-8024 Oberhaching (DE)

(56) Entgegenhaltungen:
- CHEMICAL ABSTRACTS, Band 110, 1989, Zusammenfassung Nr. 111040x, Columbus, Ohio, US; A. FOUCAULT et al.: "Study of acetic acid/formic acid/water/chloroform solvent system application to the separation of two large and hydrophobic fragments of bacteriorhodopsin membrane protein by centrifugal partition chromatography"

## Beschreibung

Die Erfindung betrifft ein Verfahren zur Herstellung von Purpurmembran enthaltend Bacteriorhodopsin.

Bacteriorhodopsin ist ein Chromoprotein, das von Halobakterien wie Halobacterium halobium gebildet wird. Es besteht aus einem Proteinteil (Bacterioopsin) und einem Chromophor (Retinal) und kommt in der Zellmembran der Halobakterien in Form der sogenannten Purpurmembran (nachfolgend auch PM genannt) vor. Bacteriorhodopsin ist in der PM in einem zweidimensional-kristallinen, hexagonalen Gitter angeordnet. Auf Grund seiner physikalischen Eigenschaften könnte Bacteriorhodopsin in Biocomputern (vgl. Biosystems 1986, 19, 223 236) oder als holographisches Speichermaterial bei der optischen Informationsverarbeitung (vgl. Biophysics 1985, 30(5), 962 - 967) eingesetzt werden. Dazu ist es allerdings nötig, große Mengen von Purpurmembranen gewinnen zu können. Auf Grund der geringen physikalischen Unterschiede von Purpurmembranen und kontaminierenden Membranfragmenten, die kein Bacteriorhodopsin enthalten, ist es schwierig, die Purpurmembran von den kontaminierenden Fragmenten abzutrennen.

Gemäß D. Oesterhelt, W. Stoeckenius, Nature 1971, 233, 149 - 154 bzw. Methods in Enzymology 1974, 31, 667 - 678 ist es bekannt, die Herstellung durch Zentrifugation der Purpurmembran in einem Saccharosedichtegradienten 0,5 bis 1,5 M bzw. von 30 bis 50 Gew.-% Saccharose durchzuführen.
B.M. Becher, J.Y. Cassim, Preparative Biochemistry 1975, 5(2), 161 - 178, beschreiben eine Zentrifugation der Purpurmembran mittels eines Saccharosestufengradienten mit Stufen von 45, 40, 38, 36 und 16 Gew.-% Saccharose.

Wegen des hohen apparativen Aufwands bei diesen Gradientenzentrifugationen, so sind beispielweise Ultrazentrifugen nötig, ist die variable Maßstabsvergrößerung bezüglich der zu erzielenden Mengen an Bacteriorhodopsin nicht möglich, wodurch nur vergleichsweise geringe Mengen an PM pro Aufarbeitung isoliert werden.
Weiter stellen die Herstellung der Saccharosegradienten und die Entfernung der eingesetzten Saccharose von der erhaltenen PM zeitintensive Arbeitsschritte dar.

Gegenstand der Erfindung ist ein Verfahren zur Herstellung von Purpurmembran enthaltend Bacteriorhodopsin, das dadurch gekennzeichnet ist, daß
a) in an sich bekannter Weise die Zellmembran von Zellen von Halobakterien gewonnen wird und
b) zur Isolierung der Purpurmembran aus der Zellmembran eine Gelfiltrationschromatographie durchgeführt wird.

Zur Herstellung der Zellmembran werden Halobakterien wie Halobacterium halobium in an sich bekannter Weise unter Bedingungen kultiviert, die die Ausbildung von Purpurmembranen begünstigen, wie beispielsweise niedriger Sauerstoffgehalt des Mediums und Licht ( siehe D. Oesterhelt, W. Stoeckenius, Methods in Enzymology 1974, 31, 667 - 678). Nach entsprechender Kultivationszeit werden die Zellen beispielsweise durch Zentrifugation geerntet. Aus der erhaltenen Zellmasse wird in an sich bekannter Weise die Zellmembran gewonnen. Bevorzugt wird eine Dialyse gegen deionisiertes Wasser und nachfolgendes Sammeln der Zellmembran durch Zentrifugation durchgeführt. Anschließend wird diese Purpurmembran enthaltende Membranfraktion vorzugsweise mit deionisiertem Wasser gewaschen.

Diese Zellmembranfraktionen werden nach dem erfindungsgemäßen Verfahren auf eine Chromatographiesäule, gefüllt mit einem Gefiltrationsmaterial, welches eine Ausschlußgrenze bezüglich Molekulargewicht von vorzugsweise 2·10⁶ bis 10⁹, insbesondere 10⁷-10⁹, aufweist, aufgetragen und vorzugsweise mit Wasser, welches eine bevorzugte, spezifische Leitfähigkeit von vorzugsweise 0,05 bis 5 µS/cm, insbesondere 0,1 bis 1,0 µS/cm, und einen pH-Wert von vorzugsweise 6,0 bis 7,5, insbesondere von 6,3 - 6,7 zeigt, bei einer Temperatur von vorzugsweise 1 bis 30 °C, insbesondere 4 bis 10°C, eluiert.

Die Länge bzw. der Durchmesser der Chromatographiesäule kann beliebig variieren und ist abhängig von der Menge der zu reinigenden Zellmembran. So haben sich beispielweise für Mengen von 10 bis 100 mg Zellmembran Säulen mit einer Länge von 50 bis 100 cm und einem Durchmesser von 1,5 bis 5 cm bewährt.

Die Menge des zu verwendenden Trennmaterials beträgt vorzugsweise 5 - 15 ml pro mg zu reinigende Zellmembran, insbesondere 9 bis 11 ml/mg.

Die Elutionsgeschwindigkeit beträgt vorzugsweise 0,25 bis 16 cm/h, insbesondere 0,5 bis 6 cm/h, wobei vorzugsweise bei einem Durckbereich von 0-5 bar Überdruck, insbesondere 0-500 mbar Überdruck gearbeitet wird.

Vorzugsweise werden Gelfiltrationsmaterialien mit einer Teilchengröße von 20 - 150 µm, insbesondere 40 - 105 µm, eingesetzt.

Die erfindungsgemäß eingesetzten Gelfiltrationsmaterialien sind solche, die auch bisher zur Gelfiltration verwendet wurden. Vorzugsweise finden vernetzte oder unvernetzte Dextrane, Agarose-Derivate oder Copolymere von Oligoethylenglycol, Glycidylmethacrylat und Pentaerythroldimethacrylat Verwendung.

Besonders bevorzugte Gelfiltrationsmaterialien sind Sephacryl S 1000 (hierbei dürfte es sich um ein eingetragenes Warenzeichen der Firma Pharmacia AB, Uppsala, Schweden handeln), BioGel A 150m (hierbei dürfte es sich um ein eingetragenes Warenzeichen der Firma BioRad Laboratories, Richmond, Kalifornien, USA handeln) und Fractogel TSK HW 75 (hierbei dürfte es sich um ein eingetragenes Warenzeichen der Firma Merck, Darmstadt, BRD handeln).

### Beispiel 1 (Vergleichsbeispiel)

13 g Zellpaste von Halobacterium halobium wurden mit 90 ml einer Lösung aus 250 g/l NaCl, 20 g/l MgSO₄.7H₂O, 3 g/l Trinatriumcitrat und 2 g/l KCl resuspendiert, mit 5 mg Desoxyribonuclease versetzt und eine Stunde gerührt. Die Zellsuspension wurde anschließend 16 Stunden gegen deionisiertes Wasser dialysiert. Das Dialysat wurde 10 Minuten bei 7600 q zentrifugiert und der Überstand zur Herstellung der Membranen 30 Minuten bei 38000 q zentrifugiert. Das erhaltene Membransediment wurde mit 80 ml Wasser mit einer spezifischen Leitfähigkeit von 0,1 µS/cm gewaschen und erneut bei 38000 q zentrifugiert. Der 20 mg PM enthaltende Niederschlag wurde mit 3 ml deionisiertem Wasser aufgenommen und auf insgesamt 5 Saccharose-Dichtegradienten von jeweils 28 ml Volumen mit einem Saccharosegehalt von 30-50 % (w/w) aufgetragen. Die Dichtegradienten wurden anschließend für 16 Stunden bei 100000 q in einer Ultrazentrifuge zentrifugiert. Die Dichtegradienten wurden dann fraktioniert, die PM-haltigen Fraktionen vereinigt und mit 2 Volumina deionisiertem Wasser verdünnt. Die verdünnte PM-Suspension wurde alsdann für 30 Minuten bei 38000 q zentrifugiert, die erhaltenen PMs nochmals mit 100 ml deionisiertem Wasser gewaschen und erneut für 30 Minuten bei 38000 q zentrifugiert. Die hieraus erhaltene Niederschlag wurde mit wenig deionisiertem Wasser aufgenommen. Es konnten 14 mg PM gewonnen werden.

### Beispiel 2

35 g Zellpaste von Halobacterium halobium wurden mit 270 ml einer Lösung aus 250 g/l NaCl, 20 g/l MgSO₄.7H₂O, 3 g/l Trinatriumcitrat und 2 g/l KCl resuspendiert, mit 15 mg Desoxyribonuclease versetzt und eine Stunde gerührt. Die Zellsuspension wurde anschließend 16 Stunden gegen deionisiertes Wasser dialysiert. Das Dialysat wurde 10 Minuten bei 7600 q zentrifugiert und der Überstand zur Herstellung der Membranen 30 Minuten bei 38000 q zentrifugiert. Das erhaltene Membransediment wurde mit 240 ml Wasser mit einer spezifischen Leitfähigkeit von 0,1 µS/cm und pH 6,7 gewaschen und erneut bei 38000 q zentrifugiert. Der 80 mg PM enthaltende Niederschlag wurde sodann mit 7 ml Wasser obiger Reinheit und pH-Wertes aufgenommen und auf eine Säule, gefüllt mit Sephacryl S 1000 von 75 cm Länge und 5,0 cm Durchmesser, die mit Wasser mit einer spezifischen Leitfähigkeit von 0,1 µS/cm und pH 6,7 äquilibriert worden war, aufgetragen. Die Elution erfolgte bei 5°C mit einer Elutionsgeschwindigkeit von 2 cm pro Stunde. Die PM-haltigen Fraktionen wurden vereinigt und die PM durch Zentrifugation für 30 Minuten bei 38000 q gewonnen. Es konnten 60 mg Purpurmembran erhalten werden. Das optische Spektrum dieser Membranen stimmte mit dem des Bacteriorhodopsins überein, es konnten keine Verunreinigungen gefunden werden.

### Beispiel 3

Es wurde wie in Beispiel 2 verfahren, jedoch betrug die Elutionsgeschwindigkeit bei der Säulenchromatographie 5,7 ml pro Stunde. Es konnten 72 mg Purpurmembranen erhalten werden entsprechend einer Ausbeute von 90% bei der Saulenchromatographie.

### Beispiel 4

14 g Zellpaste von Halobacterium halobium wurden mit 90 ml einer Lösung aus 250 g/l NaCl, 20 g/l MgSO₄.7H₂O, 3 g/l Trinatriumcitrat und 2 g/l KCl resuspendiert, mit 5 mg Desoxyribonuclease versetzt und eine Stunde gerührt. Die Zellsuspension wurde anschließend 16 Stunden gegen deionisiertes Wasser dialysiert. Das Dialysat wurde 10 Minuten bei 7600 q zentrifugiert und der überstand zur Herstellung der Membranen 30 Minuten bei 38000 q zentrifugiert. Das erhaltene Membransediment wurde mit 80 ml Wasser mit einer spezifischen Leitfähigkeit von 0,1 µS/cm gewaschen und erneut bei 38000 q zentrifugiert. Der 20 mg PM enthaltende Niederschlag wurde sodann mit 2,5 ml Wasser obiger Reinheit und pH-Wertes aufgenommen und auf eine Säule, gefüllt mit Sephacryl S 1000 von 60 cm Länge und 2,5 cm Durchmesser, die mit Wasser mit einer spezifischen Leitfähigkeit von 0,1 µS/cm und pH-Wert 6,6 äquilibriert worden war, aufgetragen. Die Elution erfolgte bei 5°C mit einer Elutionsgeschwindigkeit von 4,6 cm pro Stunde. Die PM-haltigen Fraktionen wurden vereinigt und die PM durch Zentrifugation für 30 Minuten bei 38000 q gewonnen. Es konnten 15 mg Purpurmembran erhalten werden. Das optische Spektrum dieser Membranen stimmte mit dem des Bacteriorhodopsins überein, es konnten keine Verunreinigungen gefunden werden.

### Beispiel 5

Die Versuchsdurchführung entsprach Beispiel 4, wobei die Chromatographie bei 10°C durchgeführt wurde. Es konnten 18 mg Purpurmembranen gewonnen werden, was einer Ausbeute von 80% bei der Säulenchromatographie entspricht.

### Beispiel 6

Die Versuchsdurchführung entsprach Beispiel 4, jedoch betrug die spezifische Leitfähigkeit des Wassers 0,8 µS/cm und der pH-Wert 6,5. Es wurden 16 mg PM erhalten, was einer Ausbeute von 82% bei der Säulenchromatographie entspricht.

### Beispiel 7

Die Versuchsdurchführung entsprach Beispiel 4, jedoch wurde an einer Säule, gefüllt mit Sephacryl S 1000, von 80 cm Länge und 2,5 cm Durchmesser chromatographiert. Es konnten 15 mg Purpurmembran gewonnen werden, was einer Ausbeute von 85% bei der Säulenchromatographie entspricht.

### Beispiel 8

Die Versuchsdurchführung entsprach Beispiel 4, jedoch wurde als Trennmaterial TSK HW 75 der Firma Merck, Darmstadt, BRD, verwendet. Es konnten 17 mg Purpurmembran erhalten werden. Die Ausbeute bei der Säulenchromatographie betrug 60%.

### Beispiel 9

Die Versuchsdurchführung entsprach Beispiel 4 jedoch wurde als Eluens ein 50 µm Phosphat-Puffer mit pH 6,5 verwendet. Es wurden 18 mg Purpurmembran erhalten. Die Ausbeute betrug hier 85%.

## Patentansprüche

1. Verfahren zur Herstellung von Purpurmembran enthaltend Bacteriorhodopsin aus Zellen von Halobakterien, dadurch gekennzeichnet, daß
a) in an sich bekannter Weise die Zellmembran von Zellen von Halobakterien gewonnen wird und
b) zur Isolierung der Purpurmembran aus der Zellmembran eine Gelfiltrationschromatographie durchgeführt wird.

2. Verfahren nach Anspruch 1, dadurch gekennzeichnet, daß ein Eluens mit einer spezifischen Leitfähigkeit von 0,05 - 5 µS/cm eingesetzt wird.

3. Verfahren nach Anspruch 1 oder 2, dadurch gekennzeichnet, daß ein Eluens mit einen pH-Wert von 6,0 bis 7,5 eingesetzt wird.

4. Verfahren nach einem oder mehreren der Ansprüche 1 bis 3, dadurch gekennzeichnet, daß Wasser als Eluens eingesetzt wird.

5. Verfahren nach einem oder mehreren der Ansprüche 1 bis 4, dadurch gekennzeichnet, daß die Chromatographie bei einer Temperatur von 1 bis 30 °C erfolgt.

6. Verfahren nach einem oder mehreren der Ansprüche 1 bis 5, dadurch gekennzeichnet, daß das Gelfiltrationsmaterial eine Ausschlußgrenze bezüglich Molekulargewicht von 2·10⁶ bis 10⁹ aufweist.

## Claims

1. Process for the preparation of purple membrane containing bacteriorhodopsin from halobacteria cells, characterised in that
a) the cell membrane from halobacteria cells is obtained, in a manner known per se, and
b) the material is subjected to gel filtration chromatography in order to isolate the purple membrane from the cell membrane.

2. Process according to Claim 1, characterised in that an eluent having a specific conductivity of 0.05 - 5 µS/cm is employed.

3. Process according to Claim 1 or 2, characterised in that an eluent having a pH of 6.0 to 7.5 is employed.

4. Process according to one or more of Claims 1 to 3, characterised in that water is employed as the eluent.

5. Process according to one or more of Claims 1 to 4, characterised in that the chromatography step is effected at a temperature of 1 to 30°C.

6. Process according to one or more of Claims 1 to 5, characterised in that the gel filtration material has an exclusion range with respect to molecular weight of 2 × 10⁶ to 10⁹.

## Revendications

1. Procédé pour obtenir la membrane pourpre contenant de la bactériorhodopsine à partir de cellules d'halobactéries, caractérisé en ce que
a) on obtient, par un procédé connu en soi, la membrane cellulaire à partir de cellules d'halobactéries et
b) on réalise une chromatographie par gel-filtration pour l'isolement de la membrane pourpre à partir de la membrane cellulaire.

2. Procédé selon la revendication 1, caractérisé en ce qu'on utilise un éluant d'une conductibilité spécifique de 0,05 à 5 µS/cm.

3. Procédé selon la revendication 1 ou 2, caractérisé en ce qu'on utilise un éluant d'un pH de 6,0 à 7,5.

4. Procédé selon l'une ou plusieurs des revendications 1 à 3, caractérisé en ce qu'on utilise l'eau en tant qu'éluant.

5. Procédé selon l'une ou plusieurs des revendications 1 à 4, caractérisé en ce qu'on réalise la chromatographie à une température de 1 à 30°C.

6. Procédé selon l'une ou plusieurs des revendications 1 à 5, caractérisé en ce que le matériau de gel-filtration a une limite d'exclusion relative au poids moléculaire de 2.10⁶ à 10⁹.
